# EUROPEAN PATENT APPLICATION

(11) **EP 2 330 185 A1**
(43) Date of publication of application: **08.06.2011**
(21) Application number: 10193628.4
(22) Date of filing: 03.12.2010
(51) Int. Cl.: C12N 1/20, C12N 1/22, C12N 9/42, C12P 7/10, C12P 21/02, C12R 1/145

(54) **Method for producing cellulolytic enzyme using clostridium microorganism and method for culturing and proliferating clostridium microorganism**

(30) Priority: 04.12.2009 JP 2009277079
(71) Applicant: Japan International Research Center for Agricultural Sciences, Tsukuba-shi Ibaraki 305-8686 (JP)
(72) Inventor: Mori, Yutaka, Tsukuba-shi, Ibaraki 305-8686 (JP); Kosugi, Akihiko, Tsukuba-shi, Ibaraki 305-8686 (JP)
(74) Representative: Sandmann, Wolfgang

(57) **Abstract**

A method for producing cellulolytic enzyme, where clostridium microorganisms having cellulose utilizability are cultured, wherein after utilizable carbon source is consumed by the clostridium microorganisms, addition and consumption of the carbon source are repeated periodically to accumulate the cellulolytic enzyme in a culture medium.

SEQUENCE LISTING

<110> Japan International Research fro Agricultural Science

<120> Method for producing cellulolytic enzyme by Clostridium and Method for
cultivating Clostridium

<130> P30707-EP

<140> JP2009-277079
<141> 2009-12-04

<160> 2

<170> PatentIn version 3.1

<210> 1
<211> 540
<212> DNA
<213> Clostridium thermocellum

<400> 1
gctggcggcg tgcctaacac atgcaagtcg agcggggata tacggaaggt ttaccggaag 60

tatatcctag cggcggacgg gtgagtaacg cgtgggtaac ctacctcata cagggggata 120

acacagggaa acctgtgcta ataccgcata atataacggg gcggcatcgt cctgttatca 180

aaggagaaat ccggtatgag atgggcccgc gtccgattag ctagttggtg aggtaacggc 240

tcaccaaggc gacgatcggt agccgaactg agaggttggt cggccacatt ggggctgaga 300

cacggcccag actcctacgg gaggcagcag tggggaatat tgcgcaatgg gggaaaccct 360

gacgcagcaa cgccgcgtga aggaagaagg ccttcgggtt gtaaacttct ttgattgggg 420

acgaaggaag tgacggtacc caaagaacaa gccacggcta actacgtgcc agcagccgcg 480

gtaatacgta ggtggcgagc gttgtccgga attactgggt gtaaagggcg cgtaggcggg 540

<210> 2
<211> 540
<212> DNA
<213> Clostridium thermocellum

<400> 2
gctggcggcg tgcctacaca tgcaagtcga gcggggatat acggaaggtt taccggaagt 60

atatcctagc ggcggacggg tgagtaacgc gtgggtaacc tacctcatac agggggataa 120

cacagggaaa cctgtgctaa taccgcataa cataacgggg cggcatcgtc ctgttatcaa 180

aggagaaatc cggtatgaga tgggcccgcg tccgattagc tggttggtga ggtaacggct 240

caccaaggcg acgatcggta gccgaactga gaggttggtc ggccacattg ggactgagac 300

acggcccaga ctcctacggg aggcagcagt ggggaatatt gcgcaatggg ggaaaccctg 360

acgcagcaac gccgcgtgaa ggaagaaggc cttcgggttg taaacttctt tgattgggga 420

cgaaagaagt gacggtaccc aaagaacaag ccacggctaa ctacgtgcca gcagccgcgg 480

taatacgtag gtggcgagcg ttgtccggaa ttactgggtg taaagggcgc gtaggcgggg 540

## Description

### Technical Field

The present invention relates to a method for producing cellulolytic enzyme using clostridium microorganism and a method for culturing and proliferating clostridium microorganism.

### Technical Background

Cellulosic biomasses such as bagasse, straw, chaff, mushroom waste floor, farmyard manure, and wood chips draw attention as raw material resources of energy and chemical industry, which do not exert an influence on food production. Particularly, an efficient saccharification technology is earnestly desired in conversion of cellulosic biomass into fuel ethanol and sugar being fermentation feedstock.

However, the saccharification technology of cellulosic biomass is greatly difficult in comparison with starch. This is because the cellulose being a principal component of cellulosic biomass is a persistent macromolecule polysaccharide having a rigid crystal structure.

There are three saccharification methods of cellulosic biomass: physical saccharification method, chemical saccharification method, and enzyme saccharification method.

The Physical saccharification treatment includes ball milling, oscillation milling, or steam blasting, and pressure hot water treatment, where saccharification is physically treated. Because the physical treatment requires a large amount of energy, it is often used in combination with chemical saccharification and enzyme saccharification as a pretreatment.
Although alkali and acid are utilized in the chemical saccharification treatment, acid saccharification is an ancient method. The acid saccharification includes a concentrated sulfuric acid saccharification method and a dilute sulfuric two-stage saccharification method. Because of use of sulfur, the acid saccharification method requires waste treatment and reduction of environmental burden, and it is said that there is limitations in cost reduction and efficient energy conversion.

The enzyme saccharification has merits including a light burden of waste liquid collection and treatments, equipment cost reduction of chemical-resistance equipment and others, and high sugar yield without excessive degradation, in comparison with the acid saccharification. Due to these merits, the enzyme saccharification of biomass containing much starch is practically used. However, the enzyme saccharification of cellulosic biomass is extremely difficult in comparison with starchy biomass because the cellulosic biomass includes cellulose having a crystal structure as described above, and has a complex structure where hemicellulose and lignin surround crystalline cellulose. Therefore, currently the enzyme saccharification requires pretreatments including crystal-structure breaking by physical or chemical pretreatments and combination use with a large amount of hemicellulase and cellulase, before the saccharification by enzyme.

With respect to hemicellulase and cellulase, saccharification enzyme derived from aerobic filamentous bacterium trichoderma reesei is industrially used. Therefore, trichoderma bacteria have been actively studied in the saccharification of cellulosic biomass as well (Patent document 1) .

Recently, it has been realized that some-type anaerobic microorganisms produce enzyme complex cellulosome capable of efficiently degrading cellulose.

It is found that the cellulosome has a structure where many macromolecule polysaccharide degrading enzymes are bound based on protein being a skeleton and these plural enzymes cooperate to act on the cellulose, thereby showing remarkably high degrading activity of macromolecule polysaccharide (Non-patent document 1).

As anaerobic microorganisms producing the cellulosome, clostridium microorganism is reported (Non-patent documents 1 to 6).

### [Prior Art Document]

### [Patent Document]

[Patent Document 1] JP 2007-319040 A [Non-patent Document]

[Non-patent document 1] Microbiol Mol Biol Rev. 2005 Mar; 69 (1) : 124-54
[Non-patent document 2] Current Microbiology 1983 9: 195-200
[Non-patent document 3] Appl Environ Microbiol. 1980 Sept; 40 vol.3:571-577
[Non-patent document 4] Appl Environ Microbiol. 1990 Jan;54 vol. 1:37-42
[Non-patent document 5] Appl Environ Microbiol. 1988 Jan;54 vol. 1:204-211
[Non-patent document 6] Proc Natl Acad Sci USA. 2006 Oct 31; 103 (44) :16165-16169

### Summary of the Invention

### Problem to be solved by the Invention

As the clostridium microorganisms producing the cellulosome, specifically bacteria belonging to clostridium thermocellum and clostridium cellulovorans are known. However, because these microorganisms are anaerobic, their energy acquisition is inefficient and production capacity of cellulolytic enzyme including cellulosome is low. Therefore, a technology of efficiently producing the cellulolytic enzyme containing a large amount of cellulosome is required to establish.

An object of the present invention is to provide a method for producing a large amount of cellulolytic enzymes, where clostridium microorganisms that have cellulose utilizability and secrete cellulolytic enzymes are cultured for increasing bacterial density.

### Means for Solving the Problem

Inventors of the present invention reviewed a method for producing cellulolytic enzymes using clostridium microorganisms having cellulose utilizability. Inventors discovered that in a case where a carbon source concentration in culture medium was increased, a production amount of the cellulolytic enzyme did not increase but was suppressed instead. Further, inventors continued the research and discovered that gradual addition of the carbon source to the culture medium improved the bacterial density and the production amount of cellulolytic enzyme and cellulolytic enzyme was accumulated in the culture medium. Consequently, inventors achieved the present invention.

A cellulolytic enzyme production method of the present invention is a method for producing cellulolytic enzyme where clostridium microorganisms that have cellulose utilizability and secrete a cellulolytic enzyme are cultured, wherein after an utilizable carbon source is digested by the clostridium microorganisms, addition of the carbon source is repeated to accumulate cellulolytic enzyme in a culture medium during cultivation.

Another cellulolytic enzyme production method of the present invention is a method for producing cellulolytic enzyme where clostridium microorganisms t hat have cellulose utilizability and secrete a cellulolytic enzyme are cultured, wherein after an utilizable carbon source is digested by the clostridium microorganisms, addition of the carbon source is repeated during cultivation.

In a method for culturing and proliferating clostridium microorganisms that have cellulose utilizability according to the present invention, after an utilizable carbon source is digested by the clostridium microorganisms during the culture, addition of the carbon source is repeated during cultivation.

### Effect of the Invention

In the cellulolytic enzyme production method according to the present invention, the clostridium microorganisms digest the utilizable carbon source during the culture of the clostridium microorganisms that have cellulose utilizability and secrete a cellulolytic enzyme, and subsequently addition of the carbon source is repeated. This operation enables to not only increase the bacterial density in the culture medium but also release cellulosome and/or cellulolytic enzyme that adsorb to the cellulose into the culture solution. Thus, it is possible to produce a large amount of cellulosome and/or cellulolytic enzyme.

Further, in the cellulolytic enzyme production method according to the present invention, it is possible to continue consuming oligosaccharide such as cello-oligosaccharide and monosaccharide such as glucose that remain in the culture solution because bacterial proliferation and bacterial metabolism can be maintained. Therefore, it is possible not only to maintain productivity of the cellulosome and/or cellulolytic enzyme but also to improve productivity of low molecule such as organic acid and ethanol that are produced as a by-product of the metabolic activity. In such case, the low molecule compound can be collected from the culture solution.

### Brief Description of Drawings

FIG. 1 is a graph showing change in total protein in 1ml of clostridium thermocellum culture solution in Example 1 of the present invention.
FIG. 2 is a graph showing total protein in 1ml of culture solution with clostridium thermocellum cultured for a specific period in Example 1.
FIG. 3 is a graph showing date-chronological change in enzyme activity of the clostridium thermocellum culture solution in Example 2 of the present invention. (A) shows avicelase activity, (B) shows endoglucanase activity, and (C) shows xylanase activity.
FIG. 4 is a graph showing enzyme activity of culture solution with clostridium thermocellum cultured for a specific period in Example 2. (A) shows avicelase activity, (B) shows endoglucanase activity, and (C) shows xylanase activity.
FIG. 5 is a view showing an image of SDS-PAGE of cellulosome of clostridium thermocellum in Example 3 of the present invention.
FIG. 6 is a graph showing date-chronological change in bacterial proliferation by the clostridium thermocellum culture method in Example 4 of the present invention. (A) shows a change of clostridium thermocellum ATCC27405, (B)shows a change of clostridium thermocellum JK-S14.
FIG. 7 is a graph showing change in total protein in clostridium thermocellum culture solution in Example 6 of the present invention.
FIG. 8 is a view showing an image of SDS-PAGE of cellulosome of clostridium thermocellum in Example 6.
FIG. 9 shows DNA sequence alignment of JK-S14 strain and ATCC27405 strain.
FIG. 10 shows DNA sequence alignment of JK-N44 strain and ATCC27405 strain.
FIG. 11 is a DNA sequence of JK-S14 strain.
FIG. 12 is a DNA sequence of JK-N44 strain.

### Description of Embodiments'

The present invention is described in detail based on several embodiments and examples.

### [Embodiment 1]

Embodiment 1 of the present invention is a method for producing a cellulolytic enzyme, where clostridium microorganisms that have cellulose utilizability and secrete cellulolytic enzyme are cultured. In the cultivation, after clostridium microorganisms digest an utilizable carbon source, addition of carbon source is repeated for accumulating cellulolytic enzymes in the culture medium during cultivation.

The clostridium microorganisms that have cellulose utilizability and secrete cellulolytic enzyme is microorganisms preferably having abilities to utilize cellulose and secrete cellulosome being a cellulolytic enzyme. The clostridium microorganism having cellulose utilizability and secreting cellulosome is specifically a microorganism belonging to clostridium thermocellum or clostridium cellulovorans, and is preferably clostridium thermocellum. As to a bacterial strain of the clostridium thermocellum, ATCC27405 strain, ATCC31549 strain, clostridium thermocellum JK-S14(NITE BP-627) strain, or clostridium thermocellum JK-N44(NITE BP-628) strain is specifically cited. The clostridium thermocellum JK-S14(NITE BP-627) strain and clostridium thermocellum JK-N44(NITE BP-628) strain are bacterial strains that are separated by the present inventors and available from The National Institute of Technology and Evaluation, Microorganisms Depositary Center.

Especially, the clostridium thermocellum JK-S14(NITE BP-627) strain is a preferable because amount of secretion and accumulation of the cellulolytic enzyme such as cellulosome in the culture medium is more than that of the conventional microorganisms belonging to clostridium thermocellum.

The clostridium microorganism that has the cellulose utilizability and secretes the cellulolytic enzyme is referred to as"clostridium bacteria" hereinafter in this specification.

Characteristics of the clostridium thermocellum JK-S14(NITE BP-627) strain are described below.
Rod-shaped bacterium (approximately 1.6 to 3.0µm), having sporeforming ability
Thermophilic bacterium
Growth temperature: 50 ° C to 65 ° C, optimum growth temperature 60°C
Growth pH: 6.0 to 9.0, optimum pH 7.0
Obligate anaerobic bacterium, growing in gas of N₂ or CO₂ (in case of CO₂, approximately 0.4% of sodium carbonate is added to the culture medium)
Culture medium characteristics: not growing in the culture medium containing oxygen; growing well in growth pH 7.0; and growing in cellulose or cellobiose as carbon sources. Cellulose becomes cream color and degrades in the culture medium containing the cellulose.
Sugar utilizability: having utilizability of cellulose, mannan, chitosan, cellobiose, fructose, or sorbitol
Colony morphology: Halo is formed in an agar culture medium containing cellulose and a colony is white and small. In some cases, a white viscose ring is formed around the colony after a lapse of days. Spore is formed. Preservable at -80° C.
16srRNA (16 ribosomal RNA coding sequence): showing 99% of homology with clostridium thermocellum ATCC27405 strain.

Bacteriological property of the clostridium thermocellum JK-N44 bacterial strain is described below.
Rod-shaped bacterium (approximately 1.6 to 3.0µm), having sporeforming ability
Thermophilic bacterium
Growth temperature: 55 ° C to 6 5 ° C, optimum growth temperature 60°C
Growth pH: 6.0 to 9.0, optimum pH 7.0
Obligate anaerobic bacterium, growing in gas of N₂ or CO₂ (in case of CO₂, approximately 0.4% of sodium carbonate is added to the culture medium)
Culture medium characteristics: not growing in the culture medium containing oxygen; growing well in growth pH 7.0; and growing in cellulose or cellobiose as carbon sources. Cellulose becomes cream color and degrades in the culture medium containing cellulose.
Sugar utilizability: having utilizability of cellulose, mannan, chitosan, cellobiose, fructose, or sorbitol
Colony morphology: Halo is formed in an agar culture medium containing cellulose and a colony is white and small. In some cases, a white viscose ring is formed around the colony after a lapse of days. Spore is formed. Preservable at -80° C.
16srRNA (16 ribosomal RNA coding sequence): showing 99% of homology with clostridium thermocellum ATCC27405 strain.

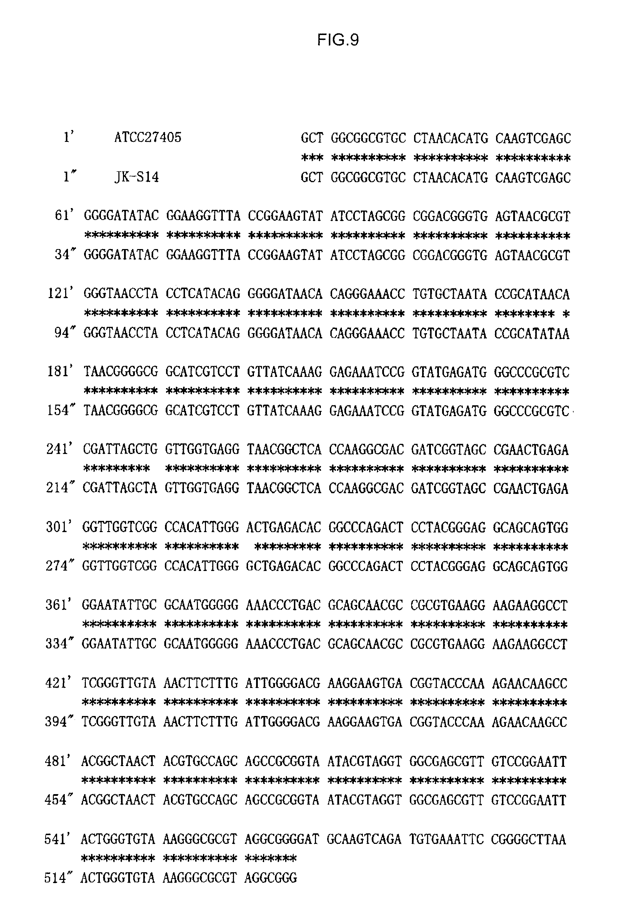

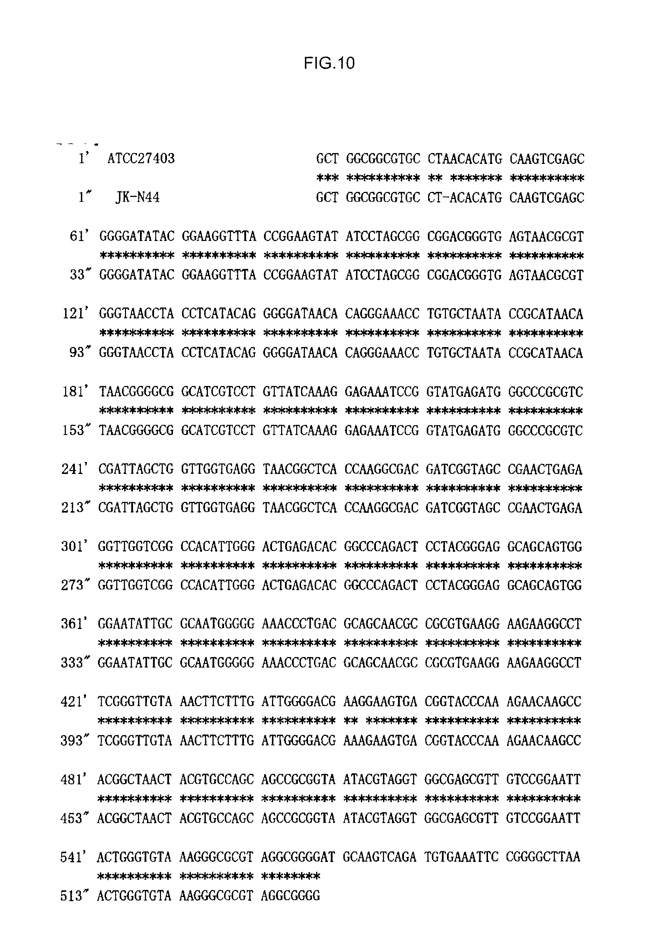

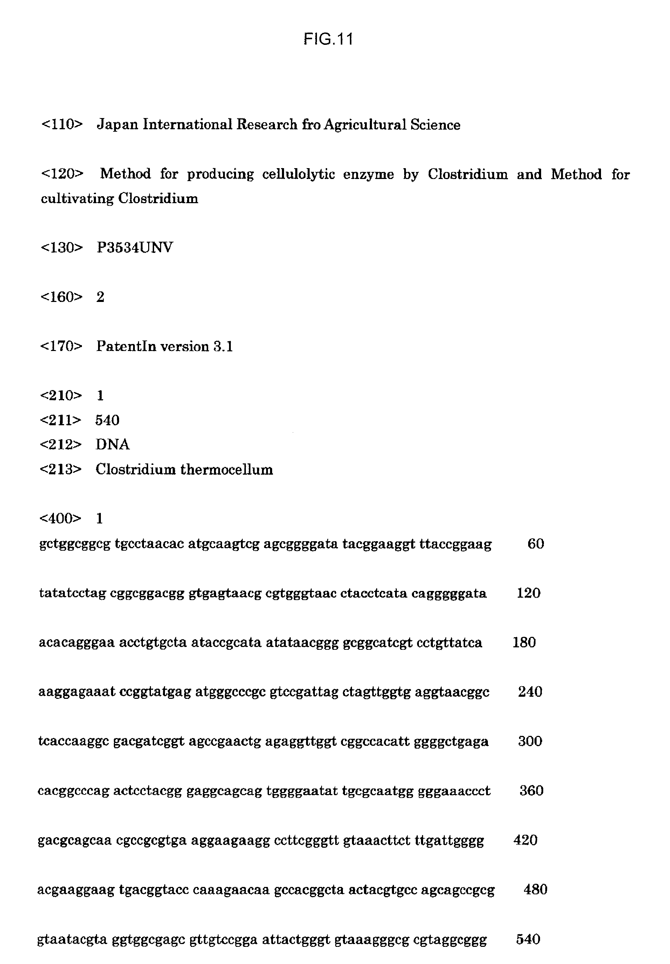

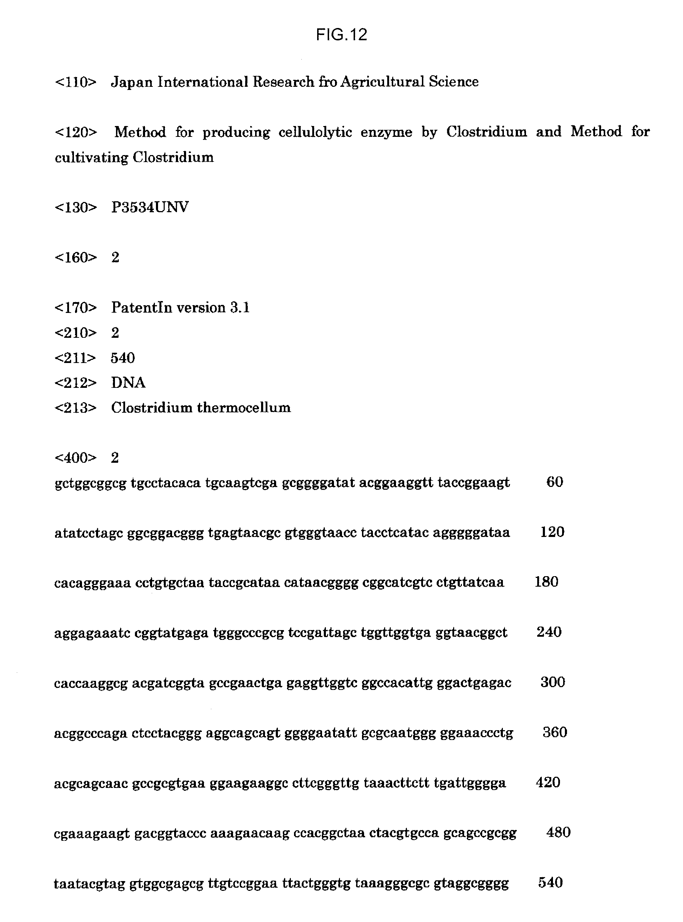

FIG. 11 shows DNA base sequence of JK-S14 strain and FIG. 12 shows DNA base sequence of JK-N44 strain. Further, FIG. 9 shows DNA sequence alignment between JK-S14 strain and ATCC27405 strain and FIG. 10 shows DNA sequence alignment between JK-N44 strain and ATCC27405 strain. FIG. 11 corresponds Sequence List NO. 1, and FIG. 12 corresponds Sequence List No. 2.

The carbon source utilizable to clostridium microorganism (hereinafter simply referred to as "carbon source" in this description) includes cellulosic substance and may be mixed with mannan, chitosan, cellobiose, fructose, and sorbitol.

Commercial cellulosic substances are available as the cellulosic substance. Specifically, whatman filter paper, chromatography cellulose powder, cotton, bacterial cellulose, and others as well as sigmacel, avicel can be used. Further, cellulose of the above cellulosic substances swelled with acid, and ones crystalline-lowered with a ball mill and crusher and recycle paper, used paper and newspaper, office waste cut paper, and toilet paper may be used as well. Further, whatever contains cellulose, such as physically or chemically pretreated rice straw, wheat straw, bagasse, wood pulp, potato pulp, used paper, paper waste, and cotton waste may be used as carbon source.

An amount of carbon source contained in the culture medium affects a production amount of cellulosome and proliferation of clostridium bacteria. A culture initial concentration and a carbon source concentration after supply of the carbon source are desired to be controlled within a range of 0.01 to 5%, preferably from 0.2 to 2%. Here, the carbon source concentration is a ratio by weight of carbon source to a culture medium volume. In a case where the carbon source concentration of the culture initial concentration or the carbon source concentration after supply of the carbon source is 0.01% or less, a consumption speed is too high and time for inducing productivity of the cellulosome is not enough. Therefore, a production yield decreases and it is not preferable. In a case where the carbon source concentration of the culture initial concentration or the carbon source concentration after supply of the carbon source are more than 5%, the proliferation of clostridium bacteria becomes slow and improvement efficiency of bacteria density in the culture medium decreases. Therefore it is not preferable. It is considered that this is because a degradation reaction speed of the carbon source by cellulosome is higher than a utilization speed of the carbon source by clostridium bacteria and reaction products accumulate excessively.

Oligosaccharide such as cellobiose and monosaccharide such as glucose that remain in the culture solution are consumed by culturing at carbon source concentration in the culture medium, not more than 0.01 to 5% or preferably less than 0.2 to 2%. Consequently, it is considered that excessive accumulation of reaction product is prevented. Further, it is possible not only to maintain productivity of cellulosome and/or cellulolytic enzyme of the clostridium bacteria, but also to release the cellulosome and/or cellulolytic enzyme that adsorb to the cellulose into the culture solution.

The amount of carbon source supplied to the culture medium is determined in consideration of timing of carbon source supply and the above-described carbon concentration in the culture medium after the carbon supply. With respect to the timing of supplying the carbon source to the culture medium, timing when the clostridium bacteria substantially consume the carbon source, in other words, when the carbon source concentration in the culture medium becomes substantially zero, is preferable. At this time, the carbon source is supplied to the culture medium each time at weight/volume ratio of less than 0.1 to 5%, preferably 0.1% to 1.5%, more preferably, 0.2 to 1%. Specific timing of supplying the cellulose, for example, may be when insoluble cellulose becomes invisible, and it is possible to be judged by observation of the culture solution. For accurate timing, the well-agitated culture solution is partly collected and filtered with a filter, and a residue amount of the cellulose and insoluble substrate can be measured. Further, a consumption amount of the cellulose and insoluble substrate can be checked by checking a precipitation amount by centrifugal separation. Further, in a case where such the check cannot be carried out, cellulose consumption can be checked when a state of proliferation of the clostridium bacteria stops, in other words, when gas generation stops, and therefore judgment for adding new substrate may be carried out at the time. In a case of the carbon source supply amount of less than 0.1 to 2% per one time, carbon source supply interval is 0.5 to 10 days. In a case of the carbon source supply amount of 0.2 to 1%, the interval is 2 to 8 days.

By supplying the carbon source at the timing when the clostridium bacteria substantially consume the carbon source, productivity of cellulolytic enzyme that begins to decrease with decrease of the carbon source concentration is recovered. This may be considered that the supply of carbon source stimulates protein expression of the clostridium bacteria.

Although a supply frequency of the carbon source is not limited, it is for example 1 to 5 times. Further, it is preferable to set up so that a total carbon supply amount obtained by multiplication of the supply frequency and the carbon source supply amount per one time is approximately 0.2 to 5%.

Although the carbon source may be individually supplied to the culture medium, it may be supplied with a part of composition of the culture medium or component of the culture medium described later.

The composition of culture medium except for the carbon source may be, for example, BM7CL culture medium composition. Further, except for the above-described culture medium component, a vitamin mixture, a trace metal mixture, and others may be added in suitable amount for stimulating the proliferation of clostridium bacteria. Mixtures of, for example, inositol, pantothenic acid, niacin, vitamin B6, vitamin B1, aminobenzoic acid, riboflavin, biotin, and folate may be added in suitable amount. Further, mixtures of element such as Fe, Na, As, Mo, K, Mn, Zn, I, Ni, Cu, and Co may be added. Further, nitrogen or nutrient source containing rich of protein hydrolysate and amino acid such as yeast extract and peptone may be added at higher concentration than the above-described culture medium concentration. Except for these, for example, ammonium sulfate, corn steep liquor, meat extract, or amino acid such as glutamic acid and glycine may be added.

Further, for suppressing pH decrease due to the proliferation, a buffering agent including inorganic salts such as phosphoric salt and citric salt, further, HEPES (2-[4-(2-Hydroxyethyl)-1-piperazinyl]ethanesulfonic acid), MOPS (3-Morpholinopropanesulfonic acid) may be added.

Culture conditions may be set up according to optimum temperature and optimum pH of the clostridium bacteria under anaerobic conditions. With respect to the anaerobic conditions, for example, a gas phase of the culture medium may be substituted by carbon dioxide gas or nitrogen gas. Because in ATCC27405 bacterium strain, SK-S14 bacterium strain, or JK-N44 bacterium strain, the optimum pH is in the vicinity of neutrality, in a case where the carbon dioxide gas is used, further lower concentration of sodium carbonate (e.g. approximately 0.4%) is preferably added to the above-described culture medium to keep pH in the vicinity of neutrality.

In the present embodiment, the carbon source is supplied to the culture medium by a semibatch culture method. During the supply of carbon source, culture medium replacement such as culture medium withdrawal is not necessary, and only adding the carbon source is also satisfied. By employing the semibatch culture method, thus produced cellulosome is accumulated in the culture medium, and it is possible to increase enzyme activity per unit amount.

As described above, according to the semibatch culture method that an operation where a small amount of the carbon source is supplied repeatedly, after the clostridium bacteria substantially consumes the carbon source, it is possible to control the carbon source concentration in the culture medium.

Then, according to this operation, a bacteria density in the culture medium is improved and a metabolic activity is also maintained without inhibiting the proliferation of clostridium bacteria. Consequently, it is possible to produce a large amount of cellulosome. Besides, because the cellulosome is accumulated in the culture medium, thus obtained cellulosome has high enzyme activity per unit amount of the culture solution. Especially, in the clostridium thermocellum JK-S14 (NITE BP-627) strain, a secretion amount and an accumulation amount of the cellulolytic enzyme such as cellulosome to the culture medium become high by the semibatch culture.

For collecting the cellulosome and/or cellulolytic enzyme from the culture medium, the culture solution may be directly concentrated by an ultrafiltration membrane and others. Protein may be precipitated using salting-out effect caused by addition of ammonium sulfate and others for collection.

It is possible to improve productivity of low molecules such as ethanol and organic acid that are produced as a by-product of metabolic activity by keeping consumption of oligosaccharide such as cello-oligosaccharide and monosaccharide such as glucose that remain in the culture solution, because bacterium proliferation and bacterium metabolism can be maintained. In such case, a low molecule compound can be collected from the culture solution.

### [Embodiment 2]

Embodiment 2 of the present invention is a method for producing cellulolytic enzyme, wherein clostridium microorganisms that have cellulose utilizability and secrete cellulolytic enzyme are cultured for producing cellulolytic enzyme, and after the clostridium microorganisms digest an utilizable carbon source, addition of the carbon source is repeated.

Instead of the semibatch method employed by Embodiment 1, Embodiment 2 employs a continuous culture method that the culture medium is replaced at the time of supply of the carbon source.

In the continuous culture method, fresh culture solution is supplied to a culture system and same amount of the culture solution is simultaneously withdrawn. This method is suitable for collecting active component secreted in the culture solution and removing harmful component. However, when new culture solution is supplied, a cellulosome/carbon source-bound unit is formed because the cellulosome is bound with the carbon source. In order to avoid isolation and collection operation of the cellulosome from the cellulosome/carbon source-bound unit, treatments may be carried out where some amount of culture solution containing cellulosome is withdrawn before new culture solution is supplied to the culture system, and the same amount of culture solution with the withdrawn amount is supplied.

For collecting the cellulosome and/or cellulolytic enzyme from thus withdrawn culture solution, the culture solution may be directly concentrated by a ultrafiltration membrane and others, or protein may be precipitated using salting-out effect caused by addition of ammonium sulfate and others. Here, again, it may be adsorbed using an absorbable cellulose carrier and degraded at an appropriate temperature, or it may be dissolved from the adsorption carrier with pure water and others for collection.

### [Embodiment 3]

Embodiment 3 of the present invention is a method for culturing and proliferating clostridium microorganisms wherein after an utilizable carbon source is digested by the clostridium microorganisms, addition of the carbon source is repeated.

The culture may be carried out under the same condition and method with those of Embodiments 1 and 2 and clostridium bacteria are separated from the culture solution by the ordinary methods.

### [Example 1]

Further, the present invention is described in detail using examples. The present invention is not limited to the examples cited below.

In order to check whether there is a change of the cellulose concentration in a total protein concentration in the culture solution during the culture, protein concentrations are measured using the batch culture method and the semibatch culture method.

Thermophilic anaerobic bacterium clostridium thermocellum ATCC27405 (American type culture collection) is used as a standard strain and clostridium thermocellum JK-S14 (NITE BP-627) is used as a test strain. For the culturing of clostridium thermocellum in BM7CO culture medium, containing 2% by weight of microcrystalline cellulose (Sigmacell type 20 produced by Sigma Aldrich Co.) in the batch culture method and 0.5% by weight at beginning in the semibatch culture method, the gas phase is replaced with highly pure carbon dioxide gas and subsequently the culture is carried out at 60°C.

The culture medium is a BM7CO culture medium composition. Specifically, potassium dihydrogen phosphate 1.5g/L, dipotassium hydrogen phosphate 2.9g/L, urea 2.1g/L, yeast extract 4.5g/L, sodium carbonate 4g/L, cysteine hydrochloride 0 . 05g/L, 0.2ml mineral solution (MgCl₂.6H₂O:5_{g}, CaCl₂.2H₂O:O.75g, FeSO₄.6H₂O:O.OO63g dissolving in 4ml of water) are prepared. Ph of the final culture medium is prepared to be approximately pH7.0.

In the semibatch culture method, an initial cellulose concentration is set 0.5%, and the above-described BM7CO culture medium including 10% cellulose is added to make a final concentration of 0.5% at a time point as an indication when the cellulose in the culture solution has no suspension degree and it is observed that the cellulose is substantially consumed (approximately 2 days). This addition operation is repeated three times to make a total value of consumption concentration 2% by weight. Further, in the semibatch culture method, for keeping the culture solution amount constant, a given amount of culture solution is sampled before the above-described BM7CO culture medium including 10% cellulose is added. The culture solution of sampling is used for analysis conducted later. Meanwhile, in the batch method as well, the sampling is conducted for the same culture days and used in a later analysis.

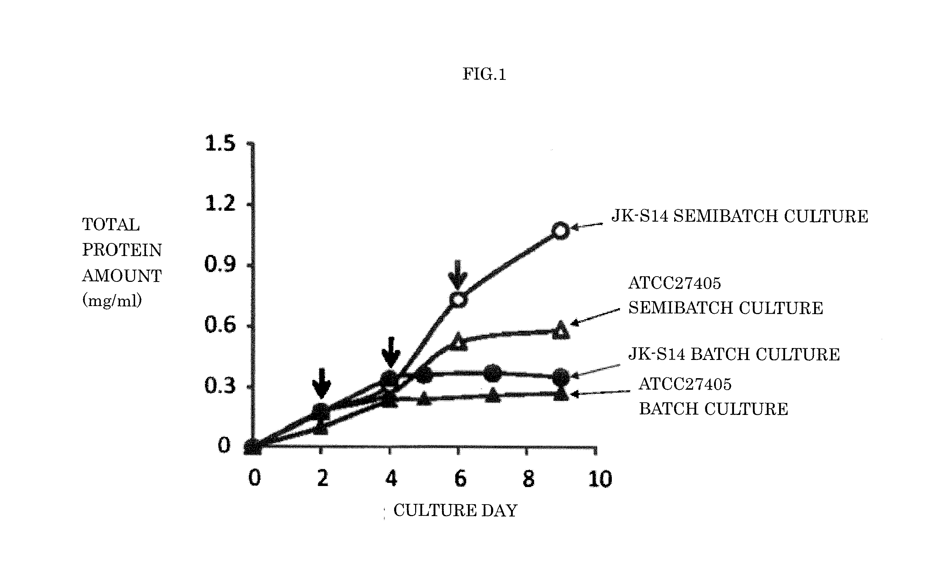

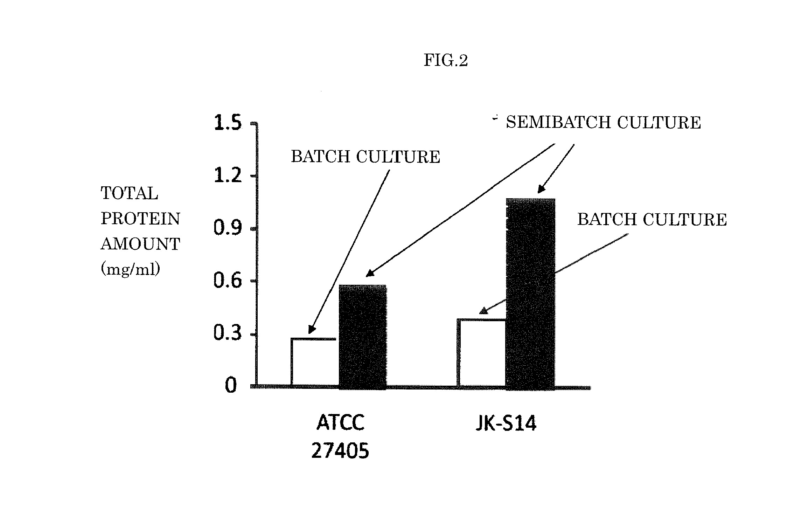

The sampled culture solution is precipitated and separated by centrifugal separation for 5 minutes at 15,000 rotation and 4°C. Culture solution supernatant and residual precipitation are preserved at -20°C. For measuring the total protein amount, this culture solution supernatant is diluted with distilled water according to necessity, and then it is measured by Bladford protein measurement kit (produced by Thermoscientific Co.). A calibration curve of protein is created using bovine serum albumin. The result is shown in FIGS. 1 and 2.

FIG. 1 shows a comparison in a total protein amount in 1 ml of culture solution between the batch culture method and the semibatch culture method that use clostridium thermocellum ATCC27405 and clostridium thermocellum JK-S14. With respect to the total protein amount in 1ml of the culture solution, a black triangle (▲) represents clostridium thermocellum ATCC27405 in the batch culture method and a black circle (●) represents clostridium thermocellum JK-S14 in the batch culture method. A white triangle (●) (▲) represents clostridium thermocellum ATCC27405 in the semibatch culture method and a white circle ( 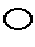 ) represents clostridium thermocellum JK-S14 in the semibatch method. Arrow marks in the figure represent culture dates when the cellulose is fed to make the concentration 0.5% after cellulose consumption of 0.5% in the semibatch culture method.

In both clostridium thermocellum ATCC27405 and clostridium thermocellum JK-S14 in the batch culture method and semibatch culture method, a change of the total protein amounts in the culture solution is not so big until the fourth day when 1% cellulose is consumed. However, after the fourth day, increase in the total protein amount is observed in the semibatch culture method. In other words, it is found that the secreted protein is difficult to be accumulated in the culture medium in the batch culture method, while it is accumulated in the culture medium by employing the semibatch culture method. Especially, in clostridium thermocellum JK-S14, remarkable increase of the total protein amount in the culture medium is observed compared with clostridium thermocellum ATCC27405. In clostridium thermocellum JK-S14, secretion amount of the protein and accumulation amount to the culture medium increase by the semibatch culture and double or more amount of clostridium thermocellum ATCC27405 is accumulated.

On the other hand, in case of the batch culture method, increase in protein production amount in the culture medium is observed in neither of bacterium strains after the fourth day of the culture. It is shown that these trends are not phenomena depending on the bacteria strain because the trends are observed in the both bacterium strains. However, the final protein amounts in 1ml culture solution of the both bacterium stains are different in the both batch culture method and the semibatch culture method at time point when 2% cellulose is consumed. In a case of batch culture method, clostridium thermocellum ATCC27405 is 0.27mg/ml, JK-S14 is 0.39mg/ml. In a case of semibatch culture method, they are 0.58mg/ml and 1.07mg/ml respectively. In other words, the semibatch culture method enables to increase protein productivity about 2 to 3 times compared with the batch culture method.

FIG. 2 shows a comparison in a final total protein amount between clostridium thermocellum ATCC27405 and clostridium thermocellum JK-S14 in the batch culture method and the semibatch culture method. It shows the total protein amount in 1ml culture solution that is sampled on the ninth day in the respective cultures. A black bar represents semibatch culture method and a white bar represents batch culture method.

In comparison with the batch culture method using clostridium thermocellum ATCC27405 and JK-S14, increase of the total protein amount in the culture solution is observed in the semibatch culture method where the cellulose is fed again after the consumption of cellulose. [Example 2]

In the batch culture method and the semibatch culture method, Microcrystal cellulolytic activity (avicelase activity), endoglucanase activity, and xylanase activity of the culture solution are measured.
In the avicelase activity, an enzyme solution is reacted at 60°C for 1 hour in 50mM sodium acetate buffer solution (pH6.0) containing 1% microcrystal cellulose powder (Sigma cell type 20 produced by Sigma Aldrich Co.) and 5mM calcium chloride, and then free sugar amount is measured by a Somogi Nelson method (Starch-related glycol enzyme experiment method 1998, Biochemical experiment method 19:41-42 published by Gakkai Shuppan Center). An amount of enzyme capable of producing 1µ mole glucose per minute is defined as 1 unit.
In the endoglucanase activity, 1% carboxymethyl cellulose (Sigma Aldrich Co. ) is used, as a substrate, instead of the cellulose powder, and then free sugar amount is measured by the Somogi Nelson method as in the avicelase activity method. An amount of enzyme capable of producing 1µ mole glucose per minute is defined as 1 unit of the endoglucanase activity.
Xylanase activity is measured as a hemicellulase activity. 1% xylan (oat spelt xylan; Sigma Aldrich Co.) is used, as a substrate, instead of the cellulose powder, and then free sugar amount is measured by Somogi Nelson method. An amount of enzyme capable of producing 1µ mole glucose per minute is defined as 1 unit of the xylanase activity as defined above.

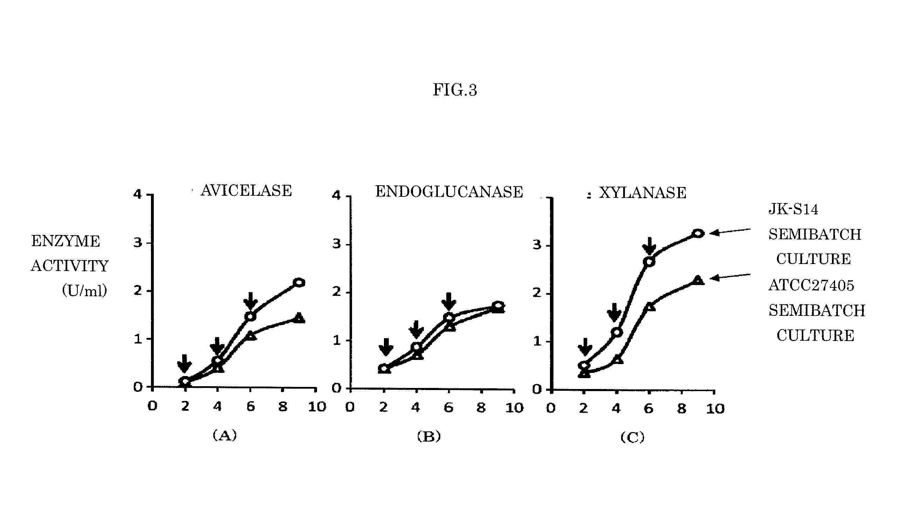

FIG. 3 (A) to FIG. 3 (C) show date-chronological activities of respective enzymes described above of the clostridium thermocellum ATCC27405 and JK-S14 in the semibatch culture method. A vertical axis represents enzyme activity and a horizontal axis represents the number of culture days.
FIG. 3(A) shows avicelase activity, FIG. 3(B) shows endoglucanase activity, and FIG. 3(C) shows xylanase activity. With respect to the enzyme activities in 1ml culture solution, a white triangle (▲) represents clostridium thermocellum ATCC27405 and a white circle ( 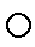 ) represents JK-S14. Arrow marks in the figure represent culture dates when the cellulose is added upon confirmation of the cellulose consumption in the semibatch culture method.

In the clostridium thermocellum ATCC27405 and JK-S14, dramatic increase in the activities of avicelase, endoglucanase, and xylanase is observed in the semibatch culture method where cellulose is added after the cellulose consumption.

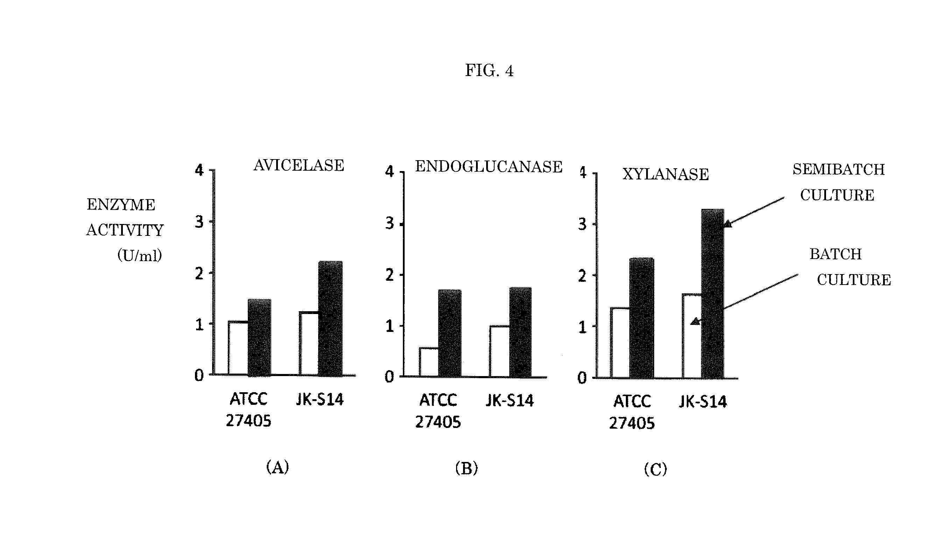

FIGs. 4(A) to 4(C) show respective enzyme activities of 1ml culture solution of clostridium thermocellum ATCC27405 and JK-S14 on the ninth day of the culture, in the batch culture method and semibatch culture method. FIG. 4(A) shows avicelase activity, FIG. (B) shows endoglucanase activity, and FIG. (C) shows xylanase activity. The black bar represents the semibatch culture method and the white bar represents the batch culture method.

In comparison among respective enzyme activities of the batch culture method in the ninth day of culture as well, the enzyme activities in the semibatch culture method are found high. Therefore, it is considered that, in the semibatch culture method, productivity of the cellulosome and other degrading enzymes increases as the protein amount increases in the culture solution. In other words, it is found that the secreted cellulolytic enzyme can be accumulated in the culture medium by the semibatch culture method.

### [Example 3]

Cellulosome in the sampled culture solution is checked. Phosphate swelled cellulose is added in the culture solution and the solution is incubated at 4°C for a night. Subsequently it is centrifuged for 5 minutes at 4°C and 14,000 rotation and the phosphate swelled cellulose adsorbing cellulosome (cellulosome/phosphate swelled cellulose fraction) is collected. This cellulosome/phosphate swelled cellulose fraction is washed with 50mM tris-hydrochloride buffer solution (pH8.0). Further, a modified polyacrylamide gel electrophoresis (SDS-PAGE) sample buffer solution is added to cellulosome/phosphate swelled cellulose fraction and heated in a boiled water bath for 5 minutes after suspension. By this operation, the cellulosome fraction is dissolved out during supernatant.

The heated solution is centrifuged for 5 minutes at 4° C and 14,000 rotation and separated into insoluble cellulose and cellulosome fraction, and the supernatant cellulosome fraction is subjected to the modified polyacrylamide gel electrophoresis (SDS-PAGE) in accordance with the conventional method. For SDS-PAGE, 4% to 20% gradient commercial polyacrylamide gel (Ato Co.) is used.

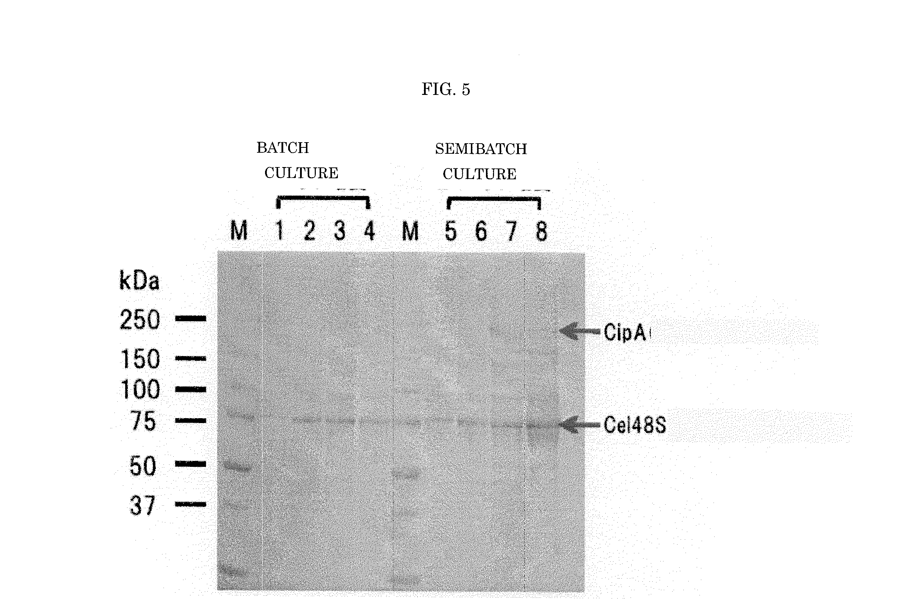

FIG. 5 shows an image of the electrophoresis of the cellulosome fraction that is prepared from 1ml culture solution of the clostridium thermocellum JK-S14 in the batch culture method and the semibatch culture method.
In the figure, M represents a protein marker, the left number represents molecular weight of the protein marker in kilo dalton. Lane 1 is a cellulosome fraction prepared from 1ml culture solution on the second day in the batch culture method. Lane 2 is a cellulosome fraction prepared from 1ml culture solution on the fourth day in the batch culture method. Lane 3 is a cellulosome fraction prepared from 1ml culture solution on the sixth day in the batch culture method. Lane 4 is a cellulosome fraction prepared from 1ml culture solution on the ninth day in the batch culture method. Lane 5 is a cellulosome fraction prepared from 1ml culture solution on the second day in the semibatch culture method. Lane 6 is a cellulosome fraction prepared from 1ml culture solution on the fourth day in the semibatch culture method. Lane 7 is a cellulosome fraction prepared from 1ml culture solution on the sixth day in the semibatch culture method. Lane 8 is a cellulosome fraction prepared from 1ml culture solution on the ninth day in the semibatch culture method.
Arrow marks of the SDS-PAGE image show estimated positions of protein bands of skeleton protein CipA being known as cellulosome-composition protein of the clostridium thermocellum and main enzyme subunit Ce148S.

The cellulosome-composition protein of the clostridium thermocellum is composed of the skeleton protein CipA and plural enzyme subunits.
The enzyme subunit Ce148S is a main enzyme subunit and is known to express in relatively large amount. Therefore, in the clostridium thermocellum JK-S14 as well, these proteins can be obviously confirmed by comparison among molecule amounts and expression amounts.
In the cellulosome fraction sample in the batch culture method, remarkable increase in proteins identified as CipA and Ce148S is not found after the fourth day. On the other hand, in the cellulosome fraction in the semibatch culture method, it is found that respective protein bands slowly increase by addition of the cellulose substrate from the second day to the fourth day. Especially, it is recognized that expression amount of the above-described cellulosome composition protein rapidly increases after the fourth day of the semibatch culture method. These results suggest that it is a phenomenon that correspond total protein amount increase in the culture solution and enzyme activity increase and many of the increased total protein are proteins derived from the cellulosome.

It is clear that the semibatch culture method where adding the cellulose is repeated in several times after consuming the cellulose is effective, especially as a culture method, for increasing cellulosome productivity in the culture solution.

### [Example 4]

For reviewing a bacterium concentration and a behavior of the product in culture solution, the bacteria concentration and the product concentration in culture solution in the semibatch culture method are measured.
With respect to the bacterium concentration, in a case of coexisting with the cellulose, the bacterium concentration cannot be accurately measured because the suspension of cellulose interferes with the measurement, so a spectrometer is not considered appropriate for use. Therefore, comparison is made by a method for directly measuring the bacterium protein concentration associated with increase in bacterium concentration.
Previously the precipitation fraction centrifuged for separation from the culture solution supernatant, in other word, the bacteria and residual cellulose fraction are washed several times with 50mM phosphate buffer solution (pH7.0), and suspended in an appropriate amount of the buffer solution. Further, the same amount of bacteriolytic solution (0.5M Sodium hydroxide, 0.5% sodium lauryl sulfate) is added and suspended well, and it is subsequently heated in the boiled water bath for 10 minutes for breaking the bacteria, and the bacterium protein is extracted. Partly using this bacterium breaking solution, measurement is performed with BCA protein measurement kit (Thermosienctific Co.). Bovine serum albumin is used for calculation of the protein concentration, and boiled with the bacteriolytic solution is used as a standard, as described above.

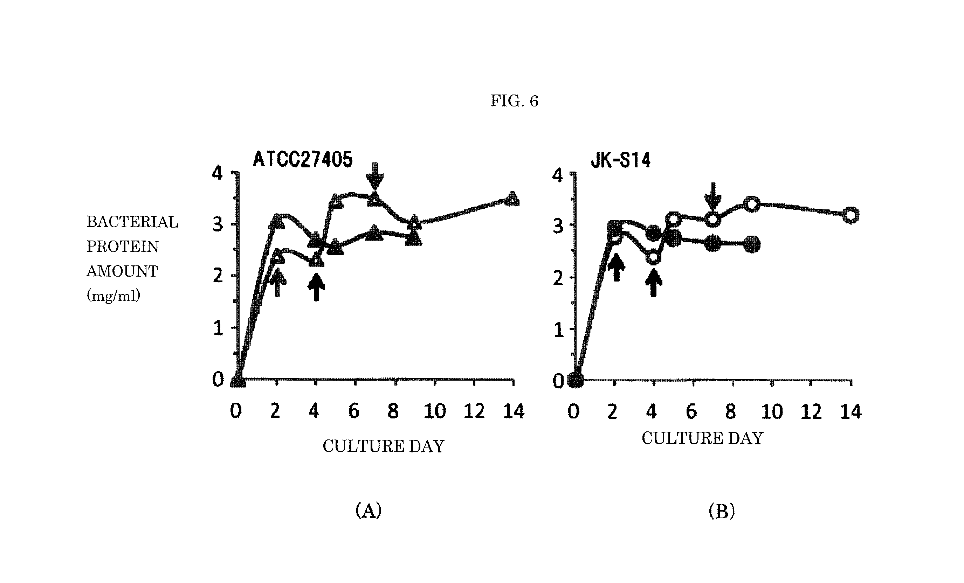

FIG. 6 shows a change in bacterium concentration in the batch culture method and the semibatch culture method.
FIG. 6(A) shows a change in bacterium concentration of clostridium thermocellum ATCC27405. A black triangle (▲) represents a change in the bacterium concentration in the batch culture method and a white triangle (▲) represents a change in the bacterium concentration in the semibatch culture method.
FIG. 6(B) shows a change in bacterium concentration of clostridium thermocellum JK-S14. A black circle(●) represents a change in the bacterium concentration in the batch culture method and a white circle (●) represents a change in the bacterium concentration in the semibatch culture method.
The bacterium protein amount in FIGs. 6(A) and 6(B) shows bacterium protein amount in 1ml culture solution. Arrow marks in the figure represent culture dates when the cellulose is added again after cellulose consumption is confirmed in the semibatch culture method.

In both clostridium thermocellum ATCC27405 and JK-S14 in the batch culture method and semibatch culture method, a change of the cell concentration is not so big until the fourth day and a big difference in bacteria concentration in early proliferation due to a difference between initial cellulose concentrations (2% in the batch culture method and 0.5% in the semibatch culture method) is not found. However, after the second addition of the cellulose, in other words, after the fourth day, increase in the bacterium concentration is found in the semibatch culture method. Further, this phenomenon shows similar trends of proliferation increase in the both bacterium strains.
These results suggest that the semibatch culture method not only simply increases the total protein productivity including cellulosome-composition protein but also causes the bacterium concentration increase.

### [Example 5]

For studying whether there are differences in the product and production amount in the culture solution between respective culture methods, constituent amounts of free sugar, organic aid, and ethanol in the culture solution are compared.
The culture solution cultured on the ninth day is used for both batch culture method and semibatch culture method. After the culture solution is centrifuged for 5 minutes at 4 °C and 14,000 rotation, supernatant is diluted with distilled water to appropriate concentration for measurement.
For measurement of the free sugar contained in the culture solution, a high-speed liquid chromatography (Shimazu Seisakusho Co. Prominence) equipped with a differential refractometer by aminex HPX-87P column (Bio-Rad) is used.
For measurement of the organic acid in the culture solution, a high-speed chromatography organic acid analysis system (Shimazu Seisakusho Co. Prominence) using post-column pH buffer electrical conductivity detection method is used.
For measurement of the ethanol in the culture solution, a gas chromatography (Shimazu Seisakusho, Model BC-2014) is used for ethanol concentration.

Table 1 shows results. Concentrations of cellobiose and glucose as the free sugar, lacto acid and acetic acid as the organic acid, and ethanol are shown.

**[Table 1]**

| CONSTITUENT (mg/ml) | ATCC27405 | | JK-S14 | |
|---|---|---|---|---|
| | BATCH CULTURE METHOD | SEMIBATCH CULTURE METHOD | BATCH CULTURE METHOD | SEMIBATCH CULTURE METHOD |
| CELLOBIOSE | 0.17 | 0.05 | 0.15 | 0.08 |
| GLUCOSE | 2.02 | 0.31 | 1.42 | 0.30 |
| LACTIC ACID | 0.35 | 0.13 | 0.21 | 0.02 |
| ACETIC ACID | 0.69 | 0.85 | 1.19 | 1.14 |
| ETHANOL | 1.00 | 2.35 | 1.05 | 2.34 |

In the culture solution subjected to the batch culture method in the clostridium thermocellum ATCC27405 and JK-S14, remarkable accumulation of the cellobiose, glucose, and lactic acid is recognized compared with the semibatch culture method. Especially, it is found that concentrations in the culture solutions of cellobiose and glucose that seem to have influence on expression of the cellulosome-related enzyme and sugar metabolic enzyme are high; concentration of the cellobiose is approximately two to three times, and glucose is approximately five to seven times, compared with those in the semibatch method.
With respect to the concentration of organic acid that is considered to affect growth, accumulation degree of the acetic acid is similar. However, concentration accumulation of the lactic acid in the batch culture method is approximately three times higher in clostridium thermocellum ATCC27405 and 10 times higher in clostridium thermocellum JK-S14, and it is considered to affect proliferation.
On the other hand, concentration increase of a produced ethanol in the semibatch culture method is approximately two times compared with the batch culture method. In the semibatch culture method, it is considered that cellobiose, glucose and lactic acid are not accumulated unlike the batch culture method, but these products are metabolized and converted to the ethanol and proliferation and metabolic activity continue and sustain after addition of the cellulose substrate.

In the semibatch culture method where after clostridium organisms digest utilizable carbon source, operation of adding the carbon source is repeated, it is considered that accumulation of cellobiose, glucose, and lactic acid is prevented. Further, based on the results of Examples 1 to 5, it is considered that the semibatch culture method stimulates and sustains the expression of cellulose related genes and maintains the bacteria proliferation to maintain the cellulosome productivity.

Generally, in culture of microorganisms, it is known that proliferation of the microorganisms is suppressed when production amount of organic acid, specifically lactic acid, increases. Based on the results of Examples 1 to 5, it is found that in the batch culture method, production amount of lactic acid increases to suppress bacterium proliferation. In the batch culture, a phenomenon is observed that a metabolic system shifts from ethanol production system to lactic acid production system when soluble concentration of oligosaccharide such as cello oligosaccharide and monosaccharide such as glucose in the culture solution increases. Therefore, it is found that the batch culture is not appropriate for a method for producing cellulolytic enzyme.

### [Example 6]

Decrease in the amount of supplied carbon source and the nutrient source is effective in culture cost and handling and practically advantageous. Therefore, in the semibatch culture method, it is studied whether increase in the cellulosome production amount is observed even in a case where addition amount of cellulose per one time is lowered. In a case where concentrations of the initial cellulose and additional cellulose are set 0.2%, cellulose is supplied four times in total, and finally 1% cellulose is consumed, total protein amount in the culture solution and cellulosome productivity are compared with the batch culture method.

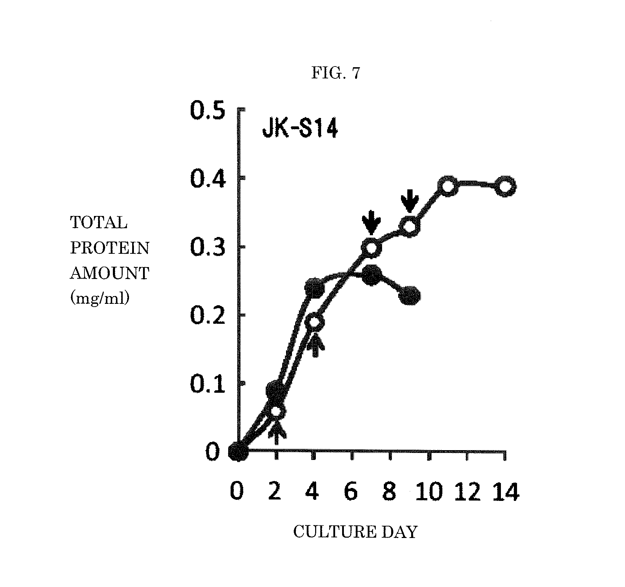

FIG. 7 shows a comparison in total protein amount of the 1ml culture solution between the batch culture method and the semibatch culture method that use clostridium thermocellum JK-S14.
A black circle(●) represents a total protein amount in the 1ml culture solution of 1% cellulose concentration in the batch culture method. A white circle ( 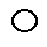 ) represents a total protein amount in the 1ml couture solution in the semibatch culture method, where the initial cellulose concentration is set 0.2%, the additional cellulose concentration per one time is set 0.2%, and supply is repeated four times. Arrow marks in the figure represent culture dates when 0.2% cellulose is added after the consumption of 0.2% cellulose in the semibatch culture method.
The total protein amount in the 1ml cellulose culture solution is measured using bovine serum albumin as standard with a Bladford protein measurement kit.

The protein production amount of protein that is accumulated in the culture solution in the batch culture method is maximum 0.28mg/ml. On the other hand, by once or twice cellulose addition in the semibatch culture method, difference in the protein amount in the culture solution between the batch method and the semibatch method is not recognized. At the cellulose concentration of 0.6% in total, the semibatch method enables to produce protein amount equal to 1% cellulose concentration in the batch culture method. Further, in the semibatch culture method, the protein concentration increases even after the third cellulose supply and finally reaches maximum 0.42mg/ml that is approximately double compared with the batch culture method.

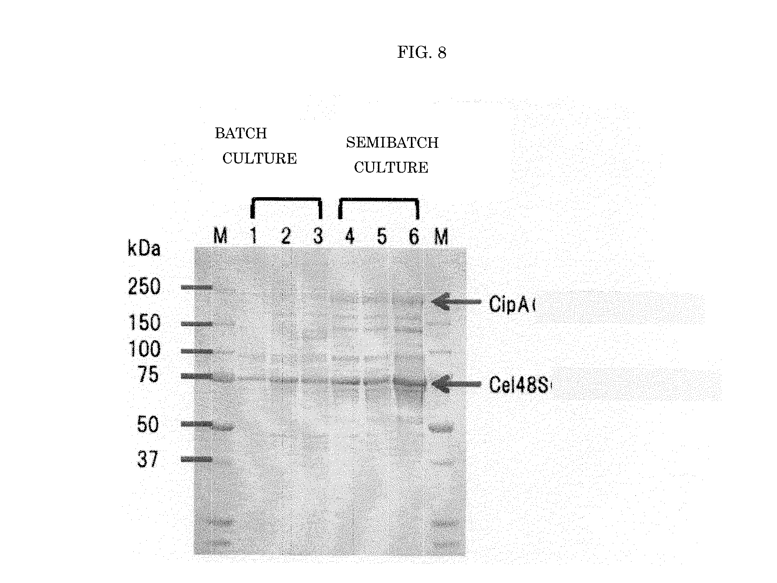

FIG. 8 shows SDS-PAGE where cellulosome productivity in the culture solution is confirmed in the semibatch culture method using the above-described clostridium thermocellum JK-S14.
M represents a protein molecular weight marker, a vertical axis represents molecular weight of the protein marker in kilo dalton. Lanes 1 to 3 are SDS-PAGE by the batch culture method and Lanes 4 to 6 are SDA-PAGE by the semibatch culture method. Lane 1 is a cellulosome fraction in the culture solution on the second day of batch culture method. Lane 2 is a cellulosome fraction in the culture solution on the fourth day of batch culture method. Lane 3 is a cellulosome fraction in the culture solution on the seventh day of batch culture method. Lane 4 is a cellulosome fraction in the culture solution that is sampled when the cellulose substrate is added on the seventh day of semibatch culture method. Lane 5 is a cellulosome fraction in the culture solution that is sampled when the cellulose substrate is added on the ninth day of semibatch culture method. Lane 6 is a cellulosome fraction in the culture solution that is sampled when the cellulose substrate is added on the fourteenth day of semibatch culture method. Arrow marks indicated in the right of the electrophoresis image show protein bands of skeleton protein CipA being known as cellulosome-composition protein of the clostridium thermocellum and main enzyme subunit Ce148S.

With respect to the cellulosome concentration in the culture solution of the clostridium thermocellum JK-S14 in the batch culture method, increase is recognized from the second day to the fourth day of the culture. However, since then, no increase is observed despite further lapse of culture days, and the result corresponds to the trend of the measurement of the total protein amount shown in FIG. 6. On the other hand, in the semibatch culture method, increase in the cellulosome concentration is found in the culture solution when the cellulose is supplied at the third time after the seventh day of culture. Until after the final cellulose supply, increase in the cellulosome is observed. Therefore, it is found that effect of the cellulosome production increase is obtained even the cellulose supplied at one time decreases.

Based on the results of Examples 1 to 6, in the culture of the clostridium thermocellum, it is found that the culture method where the supplied carbon source supplied repeatedly in several times can increase the cellulosome approximately two times to three times more than the batch culture method.

Further, in the culture of clostridium thermocellum, it is found that the culture method where the carbon source is supplied repeatedly in several times can increase the bacterium amount more than the batch culture method.

### [Deposition number of microorganism]

NITE BP-627
NITE BP-628

## Claims

1. A method for producing cellulolytic enzyme, comprising:
culturing clostridium microorganisms having abilities to utilize cellulose and to secrete a cellulolytic enzyme,
adding a utilizable carbon source after a utilizable carbon source in a culture medium is digested by the clostridium microorganisms, and
repeating addition of the utilizable carbon source to accumulate the cellulolytic enzyme in a culture medium after the utilizable carbon source in a culture medium is digested.

2. A method for producing cellulolytic enzyme, comprising:
culturing clostridium microorganisms having abilities to utilize cellulose and to secrete a cellulolytic enzyme,
adding a utilizable carbon source after a utilizable carbon source in a culture medium is digested by the clostridium microorganisms, and
repeating addition of the utilizable carbon source after the utilizable carbon source in a culture medium is digested.

3. The method for producing cellulolytic enzyme according to Claim 1 or 2, wherein a concentration of the carbon source that is contained in the culture medium is from 0.2 to 5%.

4. The method for producing cellulolytic enzyme according to claims 1 or 2, wherein the utilizable carbon source of the clostridium microorganisms is cellulosic substance.

5. The method for producing a cellulolytic enzyme according to claims 1 or 2, wherein the cellulolytic enzyme contains cellulosome.

6. The method for producing cellulolytic enzyme according to claim 5, wherein the clostridium microorganisms having ability to secrete a cellulolytic enzyme are clostridium thermocellum.

7. The method for producing cellulolytic enzyme according to claim 1, wherein the clostridium microorganisms having ability to secrete a cellulolytic enzyme are clostridium thermocellum JK-S14.

8. A method for culturing and proliferating clostridium microorganisms, comprising:
culturing clostridium microorganisms having ability to utilize cellulose,
adding utilizable carbon source after a utilizable carbon source in a culture medium is digested by the clostridium microorganisms having ability to utilize cellulose and to secrete a cellulolytic enzyme, and
repeating addition of the utilizable carbon source after the utilizable carbon source in a culture medium is digested.

9. The method for culturing and proliferating clostridium microorganisms according to claim 8, wherein a concentration of the carbon source that is contained in a culture medium after supply of the carbon source is from 0.2 to 5%.

10. The method for culturing and proliferating clostridium microorganisms according to claims 8 or 9, wherein the carbon source is cellulosic substance.

11. The method for culturing and proliferating clostridium microorganisms according to claim 10, wherein the clostridium microorganisms are clostridium thermocellum.

12. The method for culturing and proliferating clostridium microorganisms according to claim 11, wherein the clostridium thermocellum microorganisms are clostridium thermocellum JK-S14.
